(19)

Europäisches Patentamt

European Patent Office

Office européen des brevets

(11) **EP 4 119 132 A1**

(12) **EUROPEAN PATENT APPLICATION**

(43) Date of publication:
**18.01.2023 Bulletin 2023/03**

(21) Application number: **22194040.6**

(22) Date of filing: **30.11.2018**

(51) International Patent Classification (IPC):
**A61K 9/16** *(2006.01)*  **A61K 9/00** *(2006.01)*
**A61K 31/445** *(2006.01)*  **A61P 25/28** *(2006.01)*

(52) Cooperative Patent Classification (CPC):
**A61K 9/1647; A61K 9/0019; A61K 31/445;**
**A61P 25/28**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.11.2017  KR 20170163106**

(62) Document number(s) of the earlier application(s) in accordance with Art. 76 EPC:
**18882842.0 / 3 590 504**

(71) Applicant: **G2GBIO, Inc.**
**Yuseong-gu**
**Daejeon**
**34054 (KR)**

(72) Inventors:
• **LEE, Heeyong**
 **34032 Daejeon (KR)**

• **SEOL, Eunyoung**
 **34080 Daejeon (KR)**
• **YOON, Kwonhyeok**
 **34046 Daejeon (KR)**

(74) Representative: **Ricker, Mathias**
**Wallinger Ricker Schlotter Tostmann**
**Patent- und Rechtsanwälte Partnerschaft mbB**
**Zweibrückenstrasse 5-7**
**80331 München (DE)**

Remarks:
This application was filed on 06-09-2022 as a divisional application to the application mentioned under INID code 62.

(54) **SUSTAINED-RELEASE INJECTION PREPARATION CONTAINING DONEPEZIL AND PREPARATION METHOD THEREFOR**

(57)   The present invention relates to a sustained-release injectable preparation comprising biodegradable polymer microspheres containing donepezil as an active ingredient, and a method for producing the same, and a sustained-release preparation of donepezil sustained-release microspheres having a high content of donepezil and a method for producing the same. It is possible to maximize the therapeutic effect by decreasing gastrointestinal side effects frequently encountered in conventional oral administration agents and increasing patients' compliance of medicines.

[FIG. 1A]

Example 3

EP 4 119 132 A1

**Description**

**[TECHNICAL FIELD]**

The present invention relates to a biodegradable microsphere injection preparation having a high donepezil content and a good injectability and a method for producing the same.

**[BACKGROUND ART]**

Recently, the number of patients with dementia has rapidly increased due to the extension of the life span and the increase of the elderly population, and the management of the dementia patients has become a serious social problem. Dementia is a syndrome characterized by complex cognitive impairment characterized by amnesia, degenerative changes in intelligence, changes in personality, and behavioral abnormalities. This symptom is a degenerative brain disease associated with the central nervous system, which results in irreversible dysfunction in the neural network due to the slow death of the nerve cells causing degenerative diseases of the central nervous system, eventually leading to a permanent loss of the said function. The cause of dementia has not yet been clarified, and it has various pathologic and pathophysiological factors, so there is no cure for the fundamental treatment of dementia. Currently, most of the treatments for Alzheimer's disease, which are used as an indirect treatment method, are acetylcholinesterase inhibitors, which are decomposition factors of acetylcholines. Donepezil (trade name: Aricept), tacrine (trade name: Cognex) Among these are rivastigmine (trade name: Exelon), galantamine (trade name: Reminyl) and the like. Donepezil is an acetylcholinesterase (AChE) inhibitor and is widely used for the treatment of Alzheimer's disease of mild to severe severity.

Donepezil formulations, which are currently in commercial use, are in the form of tablets and are prescribed to patients with Alzheimer's disease in an oral form. In general, however, acetylcholinesterase inhibitors as oral agents have poor compliance, and are known to have adverse effects such as anxiety, nightmares, insomnia, and gastrointestinal-related side effects such as nausea, vomiting and diarrhea. It is also not easy to administer the drug orally to patients with considerably advanced dementia.

For the above reasons, studies have been actively conducted on new formulation of dementia drugs for continuously releasing dementia drugs for a long term via injections, rectal or transdermal administration.

For example, in Japanese Patent Laid-Open No. 1999-315016, suppositories for ointments and rectal administrations have been proposed when oral administration to the patients with severe dementia is difficult. However, these formulations have a problem that they are not practical for continuously administering the active ingredient over a long period of time.

In addition, there have been various proposals for percutaneous absorption formulations for treating dementia. However, in the case of frequently applying the drug once a day or two days, it may cause a burden on the skin. Also, there have been various technical problems such as a decrease in cohesive force and irregularity of skin permeation rate to develop a sustained-release transdermal formulation with a high concentration of a drug in the matrix.

Several studies have been proposed to develop sustained-release injections containing donepezil using biodegradable polymers.

Pengcheng Zhang et al. (Biomaterials, 28 (2007), 1882~1888) produced and evaluated donepezil-containing microspheres using copolymers of lactide and glycolide, which is biodegradable polymer. However, in that study, the amount of donepezil in the microspheres is as low as 13.2 %, so that there is a problem that the administered dose should be very large in order to apply the effective dose of donepezil for a long-term period to actual patients.

In another example, Dongkuk Pharmaceuticals has disclosed Korean Patent Publication No. 10-2014-0120496 for sustained-release injection using donepezil and high viscosity biodegradable polymer. It is said that it is preferable the ratio of lactide to glycolide to be 50:50 to 90:10. Also, it provides microspheres prepared by using a high viscosity polymer having a lactide and glycolide ratio of 85:15, i.e., RG858S manufactured by Evonik (having an intrinsic viscosity of 1.3 to 1.7 dL/g). Also, Korean Patent Publication No. 10-2014-0120496 discloses that it is necessary to use an insoluble salt such as xanafoate or napadisilate as a release control agent. As results, the manufacturing process will be too complicated, and safety data when the insoluble salt is injected into the human body will be necessary. Dongkuk Pharmaceutical was able to increase the drug content up to 36.1% by using this technology. However, when administering large amount of drug for long-term effect in the patients, the clogging occurs due to the nonuniformity of the particles. In addition, a lot of the residual microspheres exist in the syringe without being administered, thereby having difficulty to be administered. Moreover, nonuniformity of particle and the use of high viscosity polymer make it difficult to maintain reproducibility in commercial production, and it is not easy to obtain sustained-release microsphere injectables of uniform quality.

The CN 101 167 697 B discloses a long-acting sustained controlled release formulation of donepezil-like composition and a process for preparation. The composition is in the shape of micro-granular. According to the measurement by weight, it contains donepezil-like compounds 0.5 to 5 parts, carrier material of the sustained controlled release formulation 1 to 500 parts, and it further contains other common excipient in pharmacy. The composition is used for anti-senile dementia. The frequency of donepezil-like compounds is reduced, and the periodic time of administering medicament is prolonged, the compliance and conformability of the patient are improved, and the bioavailability and the therapeutic index of the donepezil are promoted. It discloses a plurality of processes for the preparation of long-acting sustained controlled release formulation of donepezil-like compounds.

Therefore, it is required to develop a sustained-release donepezil microsphere injectable drug having a high drug content of donepezil, stable drug release over a long period of time, a good injectability, and uniform particle size.

## [DISCLOSURE]

## [TECHNICAL PROBLEM]

It is an object of the present invention to solve the problems of the conventional donepezil preparation as described above, and to provide uniform sustained-release donepezil microspheres with a high drug content of donepezil and stable drug release over a long period of time as well as good injectability, and a method for producing the same.

## [TECHNICAL SOLUTION]

The problem of the present invention is solved by a microsphere for donepezil sustained-release injection preparation according to the independent claim 1 as well as by a method for preparing donepezil microspheres according to the independent claim 5. The dependent claims refer to further advantageous developments of the present invention.

## [ADVANTAGEOUS EFFECTS]

The donepezil sustained-release microsphere injection according to the present invention show excellent injectability and maintain the effective blood concentration of donepezil of the patient for a long period of time, thereby improving the compliance of the patient with dementia and maximizing the therapeutic effect.

## [DESCRIPTION OF THE DRAWINGS]

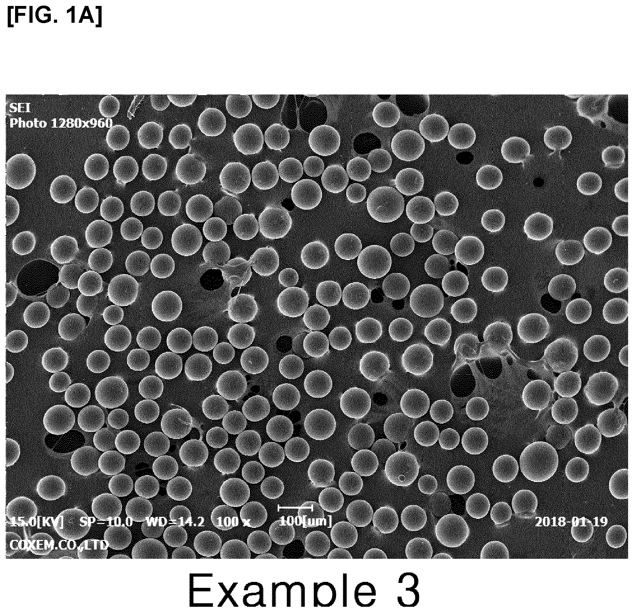

FIG. 1a is a scanning electron microscopic (SEM) photograph of the microspheres of Example 3 according to the present invention, showing that most of the microspheres have spherical morphology and have similar microsphere diameters.

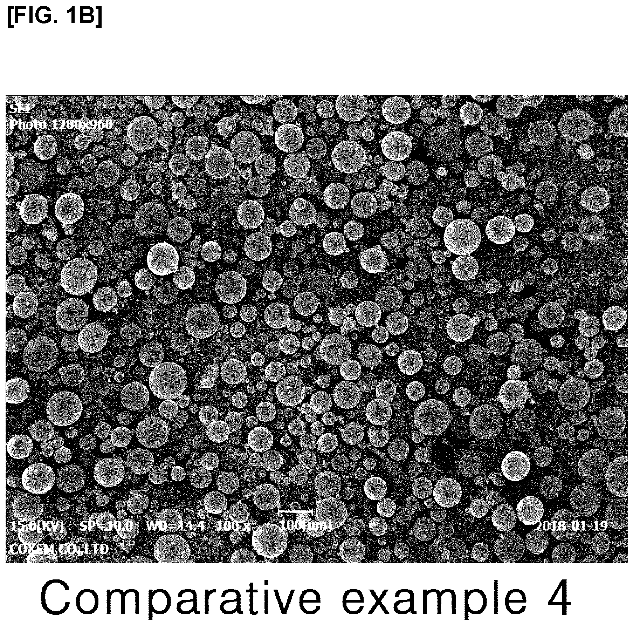

FIG. 1b is a SEM photograph of the microspheres of Comparative Example 4 prepared using a known technique, showing that most of the microspheres retained their spherical morphology but showed different morphological characteristics in terms of the size.

## [BEST MODE]

The donepezil sustained-release microsphere of the present invention is prepared using a polylactide having a lactide ratio of 100% and intrinsic viscosity of 0.16 to 0.75 dL/g.

The donepezil sustained-release microsphere of the present invention is prepared using a polylactide having a lactide ratio of 100 % as a release control agent and showing a preferred intrinsic viscosity of 0.16 to 0.75 dL/g. The intrinsic viscosity of the polylactide used in the present invention refers to that measured at 0.1 % (w/v) concentration in chloroform at 25 °C using a Ubbelohde viscometer. When the intrinsic viscosity of the polylactide is less than 0.16 dL/g, the molecular weight of the polymer is not sufficient, so that the sustained release effect of the donepezil drug may not be sufficient. When the intrinsic viscosity exceeds 0.75 dL/g, the release of donepezil may be delayed too much. In addition, there is a problem that an excessive amount of the manufacturing solvent is required and it is difficult to produce the reproducible microspheres due to the high intrinsic viscosity of the polymer for manufacturing the microsphere when using a polylactide with 0.75 dL/g more. Non-limiting examples of the commercially available polylactide polymer include Resomers R202H, R202S, R203H, R203S and R205S of Evonik Company and PDL02A, PDL02, PDL04 and PDL05 of Corbion.

The donepezil amount in the microspheres of the present invention is preferably 20 % (w/w) or more based on the total weight of the donepezil microspheres. When the amount of donepezil in the microspheres is less than 20 % (w/w), the dose required for long-term drug release becomes excessive, and the administration may be difficult. The amount of donepezil is preferably as high as possible. However, it is not desirable that the amount of donepezil is higher than 40 % (w/w) since the drug release may be too fast so that a sufficient sustained-release effect may not be obtained.

The donepezil microspheres according to the present invention preferably have an average particle size of 30 $\mu$m or more, preferably 30 to 150 $\mu$m, more preferably 35 to 150 $\mu$m, even more preferably 40 to 130 $\mu$m as well as a uniform particle distribution. The term "average particle size" used herein refers to median diameter which means a particle size corresponding to 50 % of the volume percentage in the particle size distribution curve expressed as D50 or D (v, 0.5).

If the average particle size of the donepezil microspheres is less than 30 $\mu$m, the release of the donepezil drug from the microspheres is too fast thereby undesirable. The larger the average particle size, the better the sustained-release effect of donepezil. However, if the particle size is too large, the syringe needle for injection may become too thick for patients with dementia to be administered, which may cause pain during injection. Thus, it is preferred that the average particle size of the donepezil microspheres of the present invention is 150 $\mu$m or less.

The donepezil microsphere of the present invention is characterized in having a uniform particle distribution.

In order to have an effective concentration during a long-term period by single administration, one dose of donepezil microspheres will be significantly increased. Donepezil microspheres having a uniform particle distribution can be administered in a more accurate amount with smaller deviation than the non-uniform microspheres at the time of injection. The size distribution diagram or span value of the donepezil microspheres of the present invention is preferably 1.2 or less.

More preferably, the size distribution is preferably 1.0 or less. The size distribution or span value used herein is an index indicating the uniformity of the particle size of the microspheres and is calculated by the formula of the size distribution of ((Span value) = (Dv0.9-Dv0.1) / Dv0.5).

Dv0.1 refers to the particle size corresponding to 10 % of the volume% in the particle size distribution curve of the microsphere, Dv0.5 refers to the particle size corresponding to 50 % of the volume percentage in the particle size distribution curve of the microsphere, and Dv0.9 refers to the particle size corresponding to 10 % of the volume% in the curve.

Preferably, 80 % (w/w) or more of the microspheres of the present invention may be recovered when recovering the donepezil-sustained release microspheres from the suspension of 200 mg of the microsphere of the present invention in 0.5 mL of distilled water using a 23-gauge (G) needle.

Donepezil, currently used as an oral agent, is generally administered in doses ranging from 5 mg to 10 mg per day. Assuming that the bioavailability of the oral agent and the sustained-release microspheres are similar, 150 mg to 300 mg as donepezil and 25 % (w/w) 600 mg to 1,200 mg of microspheres should be administered in a single dose in order to maintain donepezil at an effective concentration. Therefore, the high dose recovery rate of the sustained-release donepezil of the present invention may be a very important feature when applied to an actual patient.

In one specific embodiment, when the sustained-release microparticles are administered to the muscle of the SD rat, the donepezil in the microsphere release may be 0 % to 8 % in 24 hours, 20 % to 75 % in 21 days, 80 % to 100 % in 56 days, preferably 0% to 5 % in 24 hours, 25 % to 75 % in 21 days, and 80 % to 100 % in 56 days, and the donepezil release in any two weeks from administration to 56 days after administration is 5 % to 65 %, preferably 5 % to 60 %.

Hereinafter, the method for preparing the donepezil sustained-release microsphere injection of the present invention will be described in detail.

The donepezil sustained-release microsphere injection according to the present invention may be prepared, for example, by using "solvent extraction and evaporation method," but the production method is not limited thereto.

In one embodiment of the present invention, the preparation method of donepezil sustained-release microspheres is characterized by comprising:

(a) dissolving donepezil and polylactide polymer in an organic solvent to prepare a donepezil-polylactide solution (dispersed phase);
(b) adding the donepezil-polylactide solution prepared in step (a) to an aqueous phase containing a surfactant (continuous phase) to prepare an emulsion;
(c) extracting and evaporating the organic solvent from the dispersed phase in the emulsion prepared in the step (b) into the continuous phase to form microparticles; and
(d) recovering the microparticles from the continuous phase containing the microparticles of step (c) to produce donepezil microparticles.

In the step (a), the intrinsic viscosity of the polylactide is 0.10 to 1.3 dL/g, preferably 0.16 dL/g to 0.75 dL/g.

The method of homogeneously mixing the donepezil-polylactide solution and the surfactant-containing continuous phase in the step (b) is not particularly limited, but a high-speed mixer, an inline mixer, a membrane emulsion method or a microfluidic emulsion method is preferred. When an emulsion is formed using a high-speed mixer or an inline mixer, it is difficult to obtain a uniform emulsion, so that it is preferable to further perform a sieving process between steps (c) and (d) described later. When the membrane emulsion method and the microfluidics emulsion method are used, emulsions having a uniform size can be obtained. Thus, membrane emulsion method and the microfluidics emulsion method are more preferable since a sieving process and the like are not additionally required between steps (c) and (d) described later.

The kind of the surfactant used in the step (b) is not particularly limited, and any of them can be used so long as helping the donepezil-polylactide solution to form a stable liquid droplet of dispersed phase in the continuous phase. The surfactant is preferably selected from the group consisting of methylcellulose, polyvinylpyrrolidone, carboxymethylcellulose, lecithin, gelatin, polyvinyl alcohol, polyoxyethylene sorbitan fatty acid esters, polyoxyethylene castor oil derivatives and mixtures thereof. Most preferably, polyvinyl alcohol may be useful.

In step (b), the content of the surfactant in the continuous phase containing the surfactant is preferably 0.01 % (w/v) to 20 % (w/v), preferably 0.1 % (w/v) to 5 % (w/v) based on the total volume of the continuous phase. When the content of the surfactant is less than 0.01 % (w/v), a dispersed phase in the form of droplets or emulsion may not be formed in the continuous phase. When the content of the surfactant exceeds 20 % (w/v), removal of the surfactant may be difficult after the microsphere formation in the continuous phase due to excess amount of the surfactant.

In the step (c), the emulsion containing the dispersed phase in the droplet form and the continuous phase containing the surfactant is maintained or stirred at a temperature lower than the boiling point of the organic solvent for a predetermined time, for example, 2 hours to 48 hours. Then, the organic solvent may be extracted from the donepezil-polylactide solution in the form of a droplet as dispersed phase, into the continuous phase. Some of the organic solvent extracted in the continuous phase may be evaporated from the surface of continuous phase. As the organic solvent is extracted from the donepezil-polylactide solution in the droplet form and evaporated, the dispersed phase in the droplet form can be solidified to form microspheres.

In order to further efficiently remove the organic solvent in the step (c), the temperature of the continuous phase may be heated for a certain period of time.

In the step (d), the recovering donepezil microspheres may be carried out using various known techniques, for example, filtration or centrifugation.

Between step (c) and step (d), the residual surfactant may be removed by filtration and washing, and the obtained microspheres may be recovered by filtration again.

The washing step for removing the residual surfactant may be usually carried out using water, and said washing step may be repeated several times.

Further, when the emulsion is prepared by the high-speed mixer or the inline mixer in the step (b), a sieving process is additionally used to obtain uniform microspheres between step (c) and step (d). Sieving processes can be performed using known techniques. Microspheres of smaller particle size and larger particle size may be filtered using a different sieve size to obtain the microspheres with uniform particle size.

In the production method of the present invention, after the step (d) or after the filtration and washing step, the obtained microspheres may be dried using a conventional drying method to obtain the final dried microspheres.

According to the preparation method of the present invention, it is possible to produce sustained-release donepezil microsphere with a high drug content of donepezil and stable drug release over a long period of time and uniform particle size as well as good injectability.

Hereinafter, the present invention will be described in more detail with reference to the following examples. However, the following examples are illustrative of the present invention, and the present invention is not limited thereto.

**[MODE FOR INVENTION]**

**Example 1: Preparation of microspheres using PDL04 as a polymer for dispersed phase**

The dispersed phase was prepared by mixing 3.75 g of a biocompatible polymer, Purasorb PDL 04 (manufacturer: Corbion, Netherlands) and 1.25 g of donepezil base (manufacturer: Neuland Laboratories, India) with 15 g of dichloromethane (manufacturer: JT Baker, USA). The dispersed phase was sufficiently dissolved by stirring for 30 minutes or more and then used. As a continuous phase, an aqueous solution of 1 % polyvinyl alcohol (viscosity: 4.8-5.8 mPa·s) was used. A container including 1500 mL of the continuous phase was connected to an emulsification apparatus equipped with a membrane having 40 $\mu$m diameter pores, while injecting the prepared dispersed phase into the apparatus to prepare the microsphere suspension. Then the microsphere suspension was placed in a preparation vessel and stirred at a speed of 200 rpm.

Membrane emulsification apparatus and preparation vessel temperature were maintained at 25 °C. After the dispersed phase injection, the organic solvent was removed while maintaining the temperature of the microsphere suspension at 45 °C for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 1-1 Preparation of microspheres using PDL04 as a polymer for dispersed phase**

The dispersed phase was prepared by mixing 3 g of a biocompatible polymer, Purasorb PDL 04 (manufacturer: Corbion, Netherlands) and 2 g of donepezil base (manufacturer: Neuland Laboratories, India) with 12 g of dichloromethane (manufacturer: JT Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase. A container including 1200 mL of the continuous phase was connected to an emulsification apparatus equipped with a membrane having 40 $\mu$m diameter pores, while injecting the prepared dispersed phase into the apparatus to prepare the microsphere suspension. Then the microsphere suspension was placed in a preparation vessel and stirred at a speed of 200 rpm.

Membrane emulsification apparatus and preparation vessel temperature were maintained at 25 °C. After the dispersed phase injection, the organic solvent was removed while maintaining the temperature of the microsphere suspension at 45 °C for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 2: Preparation of microspheres using R202H as a polymer for dispersed phase**

The dispersed phase was prepared by mixing 3.75 g of a biocompatible polymer Resomer R202H (manufacturer: Evonik, Germany) and 1.25 g of donepezil base (manufacturer: Neuland Laboratories, India) with 9.4 g of dichloromethane (manufacturer: J.T Baker, USA). The dispersed phase was sufficiently dissolved by stirring for 30 minutes or more and then used. As a continuous phase, an aqueous solution of 1% polyvinyl alcohol (viscosity: 4.8-5.8 mPa·s) was used. A container including 940 mL of the continuous phase was connected to an emulsification apparatus equipped with a membrane having 40 $\mu$m diameter pores, while injecting the prepared dispersed phase into the apparatus to prepare the microsphere suspension. Then the microsphere suspension was placed in a preparation vessel and stirred at a speed of 180 rpm.

Membrane emulsification apparatus and preparation vessel temperature were maintained at 25 °C. After the dispersed phase injection, the organic solvent was removed while maintaining the temperature of the microsphere suspension at 42 °C for 2 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 3: Preparation of microspheres using R203H as a polymer for dispersed phase**

The dispersed phase was prepared by mixing 3.5 g of a biocompatible polymer Resomer R203H (manufacturer: Evonik, Germany) and 1.5 g of Donepezil base (manufacturer: Neuland Laboratories, India) with 9.2 g of dichloromethane (manufacturer: J.T Baker, USA). The dispersed phase was sufficiently dissolved by stirring for 30 minutes or more and then used. As a continuous phase, an aqueous solution of 1% polyvinyl alcohol (viscosity: 4.8-5.8 mPa·s) was used.

A container including 920 mL of the continuous phase was connected to an emulsification apparatus equipped with a membrane having 40 $\mu$m diameter pores, while injecting the prepared dispersed phase into the apparatus to prepare the microsphere suspension. Then the microsphere suspension was placed in a preparation vessel and stirred at a speed of 150 rpm.

Membrane emulsification apparatus and preparation vessel temperature were maintained at 25 °C. After the dispersed phase injection, the organic solvent was removed while maintaining the temperature of the microsphere suspension at 45 °C for 3 hours. After removal of the organic solvent, the temperature of the microsphere suspension was lowered to 25 °C.

The microsphere suspension was repeatedly washed several times with deionized water to remove residual polyvinyl alcohol, and the obtained microspheres were lyophilized.

**Example 4: Preparation of microspheres using R205S as a polymer for dispersed phase**

The dispersed phase was prepared by mixing 3.5 g of a biocompatible polymer, Resomer R205S (manufacturer: Evonik, Germany) and 1.5 g of Donepezil base (manufacturer: Neuland Laboratories, India) with 17.5 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase. A container including 1750 mL of the continuous phase was connected to an emulsification apparatus equipped with a membrane having 40 μm diameter pores, and a prepared dispersion was injected to prepare a microsphere suspension. Thus, the microsphere suspension was placed in a preparation vessel and stirred at a speed of 180 rpm.

The temperature of the membrane emulsification apparatus and the preparation vessel was maintained at 25 °C. After the dispersed phase injection, the organic solvent was removed while keeping the temperature of the microsphere suspension at 48 °C. for 4 hours. After removal of the organic solvent, the temperature of the microsphere suspension was lowered to 25 °C. The microsphere suspension was repeatedly washed several times with deionized water to remove residual polyvinyl alcohol, and the obtained microspheres were lyophilized.

**Example 4-1: Preparation of microspheres using R205S as a polymer for dispersed phase**

The dispersed phase was prepared by mixing 3.1 g of a biocompatible polymer, Resomer R205S (manufacturer: Evonik, Germany) and 1.9 g of donepezil base (manufacturer: Neuland Laboratories, India) with 15.5 g of dichloromethane (manufacturer: J.T Baker, USA). The dispersed phase was sufficiently dissolved by stirring for 30 minutes or more and then used. As a continuous phase, an aqueous solution of 1 % polyvinyl alcohol (viscosity: 4.8-5.8 mPa·s) was used. A container including 1550 mL of the continuous phase was connected to an emulsification apparatus equipped with a membrane having 40 μm diameter pores to prepare a microsphere suspension, while injecting the prepared dispersed phase into the apparatus to prepare the microsphere suspension. The microsphere suspension was placed in a preparation vessel and stirred at a speed of 150 rpm.

The temperature of the membrane emulsification apparatus and the preparation vessel was maintained at 25 °C. After the dispersed phase injection, the organic solvent was removed while keeping the temperature of the microsphere suspension at 48 °C. for 4 hours. After removal of the organic solvent, the temperature of the microsphere suspension was lowered to 25 °C.

The microsphere suspension was repeatedly washed several times with deionized water to remove residual polyvinyl alcohol, and the obtained microspheres were lyophilized.

**Example 4-2: Preparation of microspheres using R205S as a polymer for dispersed phase**

The dispersed phase was prepared by mixing 3.5 g of a biocompatible polymer, Resomer R205S (manufacturer: Evonik, Germany) and 1.5 g of Donepezil base (manufacturer: Neuland Laboratories, India) with 17.5 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase. A container including 1750 mL of the continuous phase was connected to an emulsification apparatus equipped with a membrane having 50 μm diameter pores, while injecting the prepared dispersed phase into the apparatus to prepare the microsphere suspension. Then the microsphere suspension was placed in a preparation vessel and stirred at a speed of 150 rpm.

The temperature of the membrane emulsification apparatus and the preparation vessel was maintained at 25 °C. After the dispersed phase injection, the organic solvent was removed while keeping the temperature of the microsphere suspension at 48 °C. for 4 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 4-3: Preparation of microspheres using R205S as a polymer for dispersed phase**

The dispersed phase was prepared by mixing 3.5 g of a biocompatible polymer, Resomer R205S (manufacturer: Evonik, Germany) and 1.5 g of Donepezil base (manufacturer: Neuland Laboratories, India) with 17.5 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase. A container including 1750 mL of the continuous phase was connected to an emulsification apparatus equipped with a membrane having 20 μm diameter pores, while injecting the prepared dispersed phase into the apparatus to prepare the microsphere suspension. The obtained microsphere suspension was placed in a preparation vessel and stirred at a speed of 150 rpm.

The temperature of the membrane emulsification apparatus and the preparation vessel was maintained at 25 °C. After the dispersed phase injection, the organic solvent was removed while keeping the temperature of the microsphere suspension at 48 °C for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 5: Preparation of microspheres using R205S as a polymer for dispersed phase**

The dispersed phase was prepared by mixing 3.5 g of a biocompatible polymer, Resomer R205S (manufacturer: Evonik, Germany) and 1.5 g of Donepezil base (manufacturer: Neuland Laboratories, India) with 17.5 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase. 1750 mL of the continuous phase was placed in a preparation vessel, and the dispersed phase was injected at a flow rate of 7 mL per minute while stirring the apparatus at a speed of 1000 rpm. When the dispersed phase injection was completed, the organic solvent was removed by stirring at 150 rpm while maintaining the preparation vessel temperature at 48 °C for 4 hours. After removal of the organic solvent, the temperature of the microsphere suspension was maintained at 25 °C.

The microsphere suspension was repeatedly washed several times with deionized water to remove residual polyvinyl alcohol and microspheres were obtained using 25 $\mu$m and 150 $\mu$m sieves. The obtained microspheres were lyophilized.

**Example 6: Preparation of microspheres using R202H and R205S as dispersed phase polymer**

The dispersed phase was prepared by mixing 1.05 g of a biocompatible polymer Resomer R202H (manufactured by Evonik, Germany), 2.45 g of Resomer R205S (manufacturer: Evonik) and 1.5 g of donepezil base (manufacturer: Neuland Laboratories, India) with dichloromethane (manufactured by JT Baker, USA). The dispersed phase was sufficiently dissolved by stirring for 30 minutes or more and then used. As a continuous phase, an aqueous solution of 1 % polyvinyl alcohol (viscosity: 4.8-5.8 mPa·s) was used. A container including 1500 mL of the continuous phase was connected to an emulsification apparatus equipped with a membrane having 40 $\mu$m diameter pores, while injecting the prepared dispersed phase into the apparatus to prepare the microsphere suspension. The microsphere suspension was placed in a preparation vessel and stirred at a speed of 200 rpm.

Membrane emulsification apparatus and preparation vessel temperature were maintained at 25 °C. After the dispersed phase injection, the organic solvent was removed while maintaining the temperature of the microsphere suspension at 45 °C. for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 7: Preparation of a formulation prepared by mixing two kinds of microsphere suspensions and hardening the same**

Preparation of dispersed phases 1 and 2 used in this experiment was carried out as follows.

Dispersed phase 1 was prepared by mixing 1.13 g of a biocompatible polymer, Resomer R202H (manufacturer: Evonik, Germany) and 0.38 g of donepezil base (manufacturer: Neuland Laboratories, India) with 2.8 g of dichloromethane (manufacturer: JT Baker, USA). The dispersed phase 2 was prepared by mixing 2.45 g of a biocompatible polymer Resomer R205S (manufacturer: Evonik, Germany) and 1.05 g of donepezil base (manufacturer: Neuland Laboratories) with 12.25 g of dichloromethane (manufacturer: JT Baker, USA). Dispersed phases 1 and 2 were used after being sufficiently dissolved by stirring for 30 minutes or more. The continuous phase was an aqueous solution of 1 % polyvinyl alcohol (viscosity: 4.8-5.8 mPa·s). A container including 282 mL of the continuous phase 1 was connected to an emulsification apparatus equipped with a membrane having 40 $\mu$m diameter pores, while injecting the prepared dispersed phase 1 into the apparatus to prepare the microsphere suspension 1. A container including 1225 mL of the continuous phase 2 was connected to an emulsification apparatus equipped with a membrane having 40 $\mu$m diameter pores, while injecting the prepared dispersed phase 2 into the apparatus to prepare the microsphere suspension 2. Temperature of both emulsification apparatuses was maintained at 25 °C. After finishing the dispersed phase injection, the microsphere suspensions 1 and 2 were collected together in a single container, stirred at a speed of 200 rpm. The organic solvent was removed while maintaining the temperature of the microsphere suspension at 45 °C for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Example 7-1: Preparation of formulations in which microspheres of Examples 2 and 4 were mixed**

The microspheres prepared in Example 2 and Example 4 were mixed at a weight ratio of 3 to 7 based on the weight of the encapsulated donepezil to prepare a formulation.

**Example 8: Preparation of a formulation prepared by mixing various microsphere suspensions**

Preparation of dispersions 1, 2 and 3 used in this experiment was carried out as follows.

Dispersion 1 was prepared by mixing 0.75 g of a biocompatible polymer Resomer R202H (manufacturer: Evonik, Germany) and 0.25 g of Donepezil base (manufacturer: Neuland Laboratories, India) with 1.88 g of dichloromethane (manufacturer: JT Baker, USA). The dispersed phase 2 was prepared by mixing 1.05 g of a biocompatible polymer Resomer R203H (manufacturer: Evonik, Germany) and 0.45 g of donepezil base (manufacturer: Neuland Laboratories, India) with 2.76 g of dichloromethane (manufacturer: JT Baker, USA). Dispersion 3 was prepared by mixing 1.75 g of biocompatible polymer Resomer R205S (manufacturer: Evonik, Germany) and 0.75 g of donepezil base (manufacturer: Neuland Laboratories, India) with 8.75 g of dichloromethane (manufacturer: JT Baker, USA). Dispersions 1, 2, and 3 were used after being sufficiently dissolved by stirring for 30 minutes or more. The continuous phase was an aqueous solution of 1 % polyvinyl alcohol (viscosity: 4.8-5.8 mPa·s).

A container including 188 mL of the continuous phase 1 was connected to an emulsification apparatus equipped with a membrane having 40 μm diameter pores, while injecting the prepared dispersed phase 1 into the apparatus to prepare the microsphere suspension 1. A container including 276 mL of the continuous phase 2 was connected to an emulsification apparatus equipped with a membrane having 40 μm diameter pores, while injecting the prepared dispersed phase 2 into the apparatus to prepare the microsphere suspension 2. A container including 875 mL of the continuous phase 3 was connected to an emulsification apparatus equipped with a membrane having 40 μm diameter pores, while injecting the prepared dispersed phase 3 into the apparatus to prepare the microsphere suspension 3. The microsphere suspensions 1, 2, and 3 were collected together in a single container, stirred at a speed of 200 rpm and the container temperature was maintained at 25 °C. Temperature of all the emulsification apparatuses was maintained at 25 °C. When the dispersed phase injection was completed, all the microsphere suspensions were collected in a single preparation vessel and stired at a speed of 200 rpm. The organic solvent was removed while maintaining the temperature of the microsphere suspension at 45 °C for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Comparative Example 1: Preparation of microspheres using PDL04 as a polymer for dispersed phase**

The dispersed phase was prepared by mixing 2.5 g of a biocompatible polymer, Purasorb PDL 04 (manufacturer: Corbion, Netherlands) and 2.5 g of donepezil base (manufacturer: Neuland Laboratories, India) with 10 g of dichloromethane (manufacturer: JT Baker, USA). The dispersed phase was sufficiently dissolved by stirring for 30 minutes or more and then used.

As a continuous phase, an aqueous solution of 1 % polyvinyl alcohol (viscosity: 4.8-5.8 m Pa·s) was used.

1000 mL of the continuous phase was loaded to an emulsification apparatus equipped with a membrane having 40 μm diameter pores, while injecting the prepared dispersion into emulsions to prepare microsphere suspension. The microsphere suspension was placed in a preparation vessel and stirred at a speed of 200 rpm.

Membrane emulsification apparatus and preparation vessel temperature were maintained at 25 °C. After the dispersed phase injection, the organic solvent was removed while maintaining the temperature of the microsphere suspension at 45 °C. for 3 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

**Comparative Example 2: Preparation of microspheres using RG858S as a polymer for dispersed phase**

The dispersed phase was prepared by mixing 3.5 g of a biocompatible polymer Resomer RG858S (manufacturer: Evonik, Germany) and 1.5 g of donepezil base (manufacturer: Neuland Laboratories, India) with 29.2 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase.

2900 mL of the continuous phase was loaded to an emulsification apparatus equipped with a membrane having

40 μm diameter pores, while injecting the prepared dispersed phase into the apparatus to prepare the microsphere suspension. The microsphere suspension was placed in a preparation vessel and stirred at a speed of 300 rpm.

Membrane emulsification apparatus and preparation vessel temperature were maintained at 25 °C. After the dispersed phase injection, the organic solvent was removed while maintaining the temperature of the microsphere suspension at 50 °C for 5 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

## Comparative Example 3: Preparation of microspheres using R205S as a polymer for dispersed phase

The dispersed phase was prepared by mixing 3.5 g of a biocompatible polymer, Resomer R205S (manufacturer: Evonik, Germany) and 1.5 g of donepezil base (manufacturer: Neuland Laboratories, India) with 17.5 g of dichloromethane (manufacturer: J.T Baker, USA). The dispersed phase was sufficiently dissolved by stirring for 30 minutes or more and then used. The continuous phase was an aqueous solution of 1 % polyvinyl alcohol (viscosity: 4.8-5.8 mPa·s), 1750 mL of the continuous phase was loaded to an emulsification apparatus equipped with a membrane having 10 μm diameter pores, while injecting the prepared dispersed phase into the apparatus to prepare the microsphere suspension. The microsphere suspension was placed in a preparation vessel and stirred at a speed of 180 rpm.

Membrane emulsification apparatus and preparation vessel temperature were maintained at 25 °C. After the dispersed phase injection, the organic solvent was removed while maintaining the temperature of the microsphere suspension at 48 °C for 4 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

## Comparative Example 3-1: Preparation of microspheres using R205S as a polymer for dispersed phase

The dispersed phase was prepared by mixing 3.5 g of a biocompatible polymer, Resomer R205S (manufacturer: Evonik, Germany) and 1.5 g of donepezil base (manufacturer: Neuland Laboratories, India) with 17.5 g of dichloromethane (manufacturer: J.T Baker, USA). The dispersed phase was used after sufficiently dissolved by stirring for 30 minutes or more. An aqueous solution of 1 % polyvinyl alcohol (viscosity: 4.8-5.8 mPa·s) was used as a continuous phase. 1750 mL of the continuous phase was loaded to an emulsification apparatus equipped with a membrane having 50 μm diameter pores, while injecting the prepared dispersed phase into the apparatus to prepare the microsphere suspension. The microsphere suspension was placed in a preparation vessel and stirred at a speed of 180 rpm.

Membrane emulsification apparatus and preparation vessel temperature were maintained at 25 °C. After the dispersed phase injection, the organic solvent was removed while maintaining the temperature of the microsphere suspension at 40 °C for 4 hours. After removal of the organic solvent, the temperature of the microsphere suspension was lowered to 25 °C.

The microsphere suspension was repeatedly washed several times with deionized water to remove residual polyvinyl alcohol, and the microspheres were lyophilized.

## Comparative Example 3-2: Preparation of microspheres using R205S as a polymer for dispersed phase

The dispersed phase was prepared by mixing 3.5 g of a biocompatible polymer, Resomer R205S (manufacturer: Evonik, Germany) and 1.5 g of donepezil base (manufacturer: Neuland Laboratories, India) with 17.5 g of dichloromethane (manufacturer: J.T Baker, USA). The dispersed phase was sufficiently dissolved by stirring for 30 minutes or more and then used. An aqueous solution of 1 % polyvinyl alcohol (viscosity: 4.8-5.8 mPa·s) was used as a continuous phase.

1750 mL of the continuous phase was loaded in the preparation vessel equipped with high speed mixer. Then, the continuous phase was stirred with the high-speed mixer at a speed of 1000 rpm, while feeding the dispersed phase at a flow rate of 7 mL. When the dispersed phase injection was completed, the organic solvent was removed by stirring at 180 rpm while maintaining the preparation vessel temperature at 48 °C for 4 hours. After removal of the organic solvent, the temperature of the microsphere suspension was maintained at 25 °C.

The microsphere suspension was repeatedly washed several times with deionized water to remove residual polyvinyl alcohol, and the microspheres were lyophilized.

## Comparative Example 4: Preparation of microspheres using R203H as a polymer for dispersed phase

The dispersed phase was prepared by mixing biocompatible polymer, Resomer R203H (manufacturer: Evonik, Germany) and 1.5 g of donepezil base (manufacturer: Neuland Laboratories, India) with 9.2 g of dichloromethane (manufacturer: J.T Baker, USA). The dispersed phase was used after sufficiently dissolved by stirring for 30 minutes or more. The continuous phase was an aqueous solution of 1 % polyvinyl alcohol (viscosity: 4.8-5.8 mPa·s). 920 mL of the continuous phase was loaded in the preparation vessel equipped with high speed mixer. Then, the continuous phase was stirred with the high-speed mixer at a speed of 1000 rpm, while feeding the dispersed phase at a flow rate of 7 mL. When the dispersed phase injection was completed, the organic solvent was removed by stirring at 150 rpm while maintaining the preparation vessel temperature at 45 °C for 3 hours. After removal of the organic solvent, the temperature of the microsphere suspension was maintained at 25 °C.

The microsphere suspension was repeatedly washed several times with deionized water to remove residual polyvinyl alcohol, and the obtained microspheres were lyophilized.

**Experimental Example 1: Measurement of donepezil dose in the microspheres**

In order to measure the donepezil content of microspheres of Examples and Comparative examples, 10 mg of the microspheres were completely dissolved with DMSO and then diluted with the mobile phase. 20 $\mu$L of the diluted solution was injected into the HPLC and the content of donepezil was measured at a detection wavelength of 271 nm. The columns used in this experimental example were Inertsil ODS-3, 5 $\mu$m, 4.6 $\times$ 150 mm, and the mobile phase used was prepared by mixing phosphate buffer (pH 5.0) and acetonitrile in a 6:4 ratio (v/v). Donepezil content of microspheres were shown in Table 1.

[Table 1]

|  | Donepezil content (%, w/w) |
|---|---|
| Example 1 | 24.6 |
| Example 1-1 | 32.9 |
| Example 2 | 27.1 |
| Example 3 | 25.4 |
| Example 4 | 27.5 |
| Example 4-1 | 34.5 |
| Example 4-2 | 27.3 |
| Example 4-3 | 27.2 |
| Example 5 | 28.1 |
| Example 6 | 26.1 |
| Example 7 | 27.3 |
| Example 7-1 | 27.3 |
| Example 8 | 26.8 |
| Comparative example 1 | 46.0 |
| Comparative example 2 | 35.8 |
| Comparative example 3 | 25.6 |
| Comparative example 3-1 | 27.6 |
| Comparative example 3-2 | 27.5 |
| Comparative example 4 | 26.2 |

As can be seen in Table 1 above, all Examples and Comparative Examples are microspheres containing at least 24.6 % (w/w) to at most 46.0 % (w/w) donepezil. Accordingly, it was found that polylactide and poly (Lactide-co-glycolide) were all usable as microsphere matrix material.

In particular, as shown in Table 1, Example 1, Example 1-1, and Comparative Example 1, in which only the initial amount of donepezil is different thereamong, show increase of the content of donepezil as the initial dose of donepezil increases.

**Experimental Example 2: Morphologic analysis by electronic microscope**

In order to analyze the morphological characteristics of the prepared microspheres, the experiment by using scanning electron microscopy was conducted.

5 mg of microspheres were placed on an aluminum stub with a carbon tape attached thereto and coated with platinum using ION-COATER (COXEM, Korea). The aluminum stub was mounted on a scanning electron microscope (COXEM EM-30, Korea) and the morphological characteristics of the microspheres were observed at an accelerating voltage of 15 kV.

As a result, as shown in FIG. 1A, most of the microspheres prepared in Example 3 tend to have a spherical shape. Further, it was confirmed that they have similar microsphere diameters.

On the other hand, according to FIG. 1B, the microspheres of Comparative Example 4 prepared using the known technique also maintained spherical shape, but showed morphological characteristics with different particle sizes.

**Experimental Example 3: Particle size analysis using laser diffraction method**

In this experiment, the average particle size, distribution and uniformity of the prepared microspheres were quantitatively measured. The experimental procedure is as follows.

50 mg of microspheres were mixed with 1 mL of deionized water by using a vortex mixer for 20 seconds, and dispersed in an ultrasonic generator for 1 minute. The microsphere dispersion was loaded in a particle size analyzer (Microtrac Bluewave, Japan) and measured for 20 seconds.

The span value as an index of particle size uniformity was obtained by the following equation (1).

[Equation 1]

$$\text{Span Value} = (D_{v,0.9} - D_{v,0.1}) / D_{v,0.5}$$

[Table 2]

|  | $D_{v,0.5}$ ($\mu$m) | Span Value |
|---|---|---|
| Example 1 | 82.0 | 0.61 |
| Example 1-1 | 86.9 | 0.59 |
| Example 2 | 74.6 | 0.69 |
| Example 3 | 81.5 | 0.63 |
| Example 4 | 79.8 | 0.60 |
| Example 4-1 | 78.7 | 0.60 |
| Example 4-2 | 126.4 | 0.66 |
| Example 4-3 | 44.2 | 0.63 |
| Example 5 | 113.9 | 0.98 |
| Example 6 | 63.3 | 0.57 |
| Example 7 | 74.8 | 0.59 |
| Example 7-1 | 74.4 | 0.59 |
| Example 8 | 79.3 | 0.63 |
| Comparative example 1 | 88.9 | 0.57 |
| Comparative example 2 | 83.1 | 0.63 |
| Comparative example 3 | 23.0 | 0.57 |
| Comparative example 3-1 | 167.0 | 0.72 |
| Comparative example 3-2 | 121.7 | 1.77 |
| Comparative example 4 | 79.3 | 1.24 |

As shown in Table 2 above, it is confirmed that the average particle size of all Examples and Comparative Examples is 30 to 150 μm except for Comparative Example 3 and Comparative Example 3-1. In particular, based on the average particle size results of Example 4, Example 4-2, Example 4-3, Comparative example 3 and Comparative example 3-1, it was confirmed that the average particle size can be controlled by varying the pore size of the membrane mounted on the membrane emulsification apparatus.

In addition, Example and Comparative example, except Example 5, Comparative example 3-2, and Comparative example 4, show a span value of 1.0 or less, and it is confirmed that they have higher particle homogeneity than the microspheres manufactured using the known technology.

The results of Example 5 confirm that it is possible to produce microspheres with a span value of 1.0 or less even when adding a sieving process that selects only the desired particles to the known technology.

## Experimental Example 4: Test of injectability of the microspheres

In order to determine the preferred microsphere average particle size through the measurement the recovery rate of the microspheres, the following experimental was conducted.

200 mg of microspheres were weighed into a 1.5 mL vial and mixed with 0.5 mL of deionized water. The microsphere suspension was recovered as much as possible using a 1 mL syringe with a 23-gauge (G) needle and then dried in a 1.5 mL vial to determine the weight of the unrecovered microspheres. The recovery rate was calculated by dividing the weight of the microspheres except for the measured non-recovered microsphere weight by the initial microsphere amount of 200 mg.

[Table 3]

|  | Recovery rate (%) |
|---|---|
| Example 3 | 94.6 |
| Example 4 | 94.2 |
| Example 4-2 | 81.5 |
| Example 4-3 | 95.1 |
| Example 5 | 86.3 |
| Comparative example 3 | 94.7 |
| Comparative example 3-1 | 5.3 |
| Comparative example 3-2 | 39.9 |

As shown in Table 3 above, Example 3, Example 4, Example 4-2, Example 4-3, Example 5, and Comparative example 3 have a particle size of 150 μm or less and a span value of 1.2 or less. The recovery rate conducted by using a syringe with 23-gause (G) needle was 86.3 % (w/w) and 95.1 % (w/w).

However, even with a low span value as in Comparative example 3-1, the microspheres having 150 μm more of the average particle size were not collected smoothly since the microspheres blocked the syringe needle. Also, the microsphere having 150 μm of the average particle size and 1.2 or more of the span value as in Comparative example 3-2, the microsphere size is uneven and the microsphere recovery was less than 50 % (w/w).

As a result, both the particle size and the span value influenced the injectability. Especially, it was found that the injectability of the microspheres with the particle size was 150 μm or less and the span value was 1.2 or less, was superior to the injectability of the other microspheres.

## Experimental Example 5: Long-term in vitro dissolution test

In order to evaluate the drug delivery capacity of sustained-release donepezil microspheres of the present invention, in vitro dissolution test of donepezil was conducted. The experimental procedure is as follows.

5 mg of microspheres and HEPES buffer (pH 7.4) were placed in a 50 mL wide-mouth bottle and stored at 37 °C in an incubator. 1 mL of the solution was taken from the bottle at the pre-set time intervals and the same amount of HEPES buffer was added. The collected solution was filtered with a 0.45 μm syringe filter and then 20 μL of the solution was injected into the HPLC. The HPLC column and operating conditions are the same as those in Example 1.

[Table 4]

| | In-vitro cumulative dissolution rate (%) of donepezil | | | | | | | | | |
|---|---|---|---|---|---|---|---|---|---|---|
| | Day 1 | Day 7 | Day 14 | Day 21 | Day 28 | Day 35 | Day 42 | Day 49 | Day 56 | Day 63 |
| Example 1 | 0.4 | 5.3 | 18.3 | 45.9 | 66.8 | 80.1 | 88.0 | 92.1 | 93.8 | 94.9 |
| Example 1-1 | 1.2 | 9.6 | 27.5 | 59.5 | 76.8 | 87.3 | 97.6 | | | |
| Example 2 | 0.3 | 6.0 | 34.3 | 69.4 | 86.6 | 93.0 | 96.5 | 98.2 | 99.2 | |
| Example 3 | 0.6 | 7.1 | 23.2 | 54.1 | 76.7 | 89.6 | 95.5 | 97.8 | 99.1 | |
| Example 4 | 0.32 | 3.6 | 7.4 | 21.0 | 40.0 | 58.3 | 72.2 | 76.6 | 78.7 | 79.2 |
| Example 4-1 | 1.1 | 8.5 | 37.8 | 62.6 | 84.5 | 94.1 | 96.0 | 98.4 | 99.5 | |
| Example 4-3 | 0.9 | 6.2 | 21.9 | 43.1 | 66.5 | 78.2 | 83.3 | 84.1 | 84.2 | 84.2 |
| Example 6 | 0.2 | 5.7 | 7.2 | 29.8 | 63.5 | 81.3 | 93.4 | 98.6 | 99.4 | |
| Example 7 | 0.3 | 4.2 | 14.7 | 39.8 | 61.2 | 80.9 | 91.4 | 96.4 | 98.7 | |
| Example 7-1 | 0.3 | 4.1 | 14.4 | 39.9 | 62.1 | 80.2 | 92.0 | 96.6 | 98.6 | |
| Com parative example 1 | 4.2 | 37.3 | 90.2 | 95.6 | 96.1 | | | | | |
| Com parative example 2 | 10.2 | 75.3 | 88.1 | 97.3 | 98.8 | 99.2 | | | | |
| Com parative example 3 | 6.5 | 19.8 | 66.3 | 87.2 | 96.8 | 98.9 | 99.4 | | | |

As shown in Table 4, Example 1, Example 1-1 and Comparative Example 1 which were prepared under the same conditions except for the donepezil amount, show that their cumulative dissolution rate rapidly increased as the amount of donepezil increased. In particular, Comparative example 1 shows 90.2 % of the drug dissolution during 14 days, thus it was unsuitable as a sustained-release microsphere. Therefore, it was determined that the donepezil amount suitable for the sustained-release microspheres was at least 20 % (w/w) to at most 40 % (w/w).

As shown in the results of Comparative example 2, the encapsulation of donepezil using poly (lactide-co-glycolide) was not difficult but poly (lactide-co-glycolide) was not suitable for sustained-release microspheres due to relatively fast dissolution.

In case of Comparative example 3 with the average particle size below 30 $\mu$m, the surface area of the microspheres is widened, so that the water decomposition of the polymer is accelerated and the encapsulated donepezil is eluted in a shorter time than the particles larger than 30 $\mu$m.

Thus, as shown in Table 4 above, all the samples showed a dissolution rate of less than 60 % within 14 days, while the comparative example was at least 60 %. Thus, it was found that the microspheres using the polylactide polymer having an intrinsic viscosity of 0.16 to 0.75 dL/g, (20~40 % (w/w)) and appropriate average particle size (30~150 $\mu$m) were found to be suitable for sustained-release microspheres.

In addition, Example 6 and Example 7 show that both of the microspheres prepared by using mixed polylactide having an intrinsic viscosity of 0.16 to 0.75 dL/g, and the microspheres wherein each microsphere was prepared separately with different polylactide having an intrinsic viscosity of 0.16 to 0.75 dL/g and mixed shows long-term donepezil dissolution from the microspheres.

**Experimental Example 6: Single-dose subcutaneous pharmacokinetic test using Sprague-Dawley rats**

In order to evaluate the potential for the donepezil microspheres of the present invention as the sustained-release treatment, the donepezil concentration in rat plasma was measured.

The microspheres were measured (86.8 mg/kg as donepezil) and dispersed in a 0.3 mL suspension, followed by intramuscular injection into SD rats. 0.25-0.5 mL of blood samples were collected at predetermined intervals and donepezil blood concentrations were measured using HPLC.

[Table 5]

| | Cumulative release rate of blood donepezil (%) | | | | | | | | | |
|---|---|---|---|---|---|---|---|---|---|---|
| | Day 1 | Day 7 | Day 14 | Day 21 | Day 28 | Day 35 | Day 42 | Day 49 | Day 56 | Day 63 |
| Example 4 | 0.3 | 3.0 | 7.1 | 26.3 | 44.2 | 74.8 | 89.7 | 94.4 | 98.1 | 100.0 |

(continued)

| | Cumulative release rate of blood donepezil (%) | | | | | | | | | |
|---|---|---|---|---|---|---|---|---|---|---|
| | Day 1 | Day 7 | Day 14 | Day 21 | Day 28 | Day 35 | Day 42 | Day 49 | Day 56 | Day 63 |
| Example 4-1 | 3.7 | 11.4 | 23.2 | 58.4 | 86.1 | 90.2 | 93.8 | 96.7 | 99.9 | 100.0 |
| Example 6 | 0.3 | 3.2 | 8.9 | 25.5 | 55.6 | 77.8 | 88.9 | 94.8 | 98.1 | 100.0 |
| Example 7 | 0.5 | 5.2 | 16.7 | 32.0 | 53.6 | 75.3 | 88.6 | 95.0 | 98.2 | 100.0 |
| Example 7-1 | 0.6 | 5.2 | 17.1 | 33.2 | 54.1 | 75.7 | 88.5 | 94.8 | 97.9 | 100.0 |
| Example 8 | 0.4 | 4.7 | 18.5 | 39.2 | 59.6 | 81.8 | 93.7 | 96.7 | 98.8 | 100 |
| Com parative example 2 | 11.6 | 88.5 | 98.4 | 98.9 | 99.2 | 99.5 | 99.9 | 100.0 | 100.0 | 100.0 |

As shown in Table 5 above, unlike Comparative example 2, the cumulative release rate of all sample microspheres was less than 5 % for one day. If the initial release is high, it may lead to a rapid increase in serum concentration of donepezil and it may be toxic, so it is not suitable for sustained release microspheres.

Also, long-term plasma donepezil concentration could be maintained only when the cumulative release rate was less than 60 % within 14 days after the administration. Moreover, a cumulative release of more than 25 % for at least 21 days and more than 80 % for 56 days after the administration is sufficient to maintain adequate blood donepezil level.

According to the results of Example 6, Example 7, Example 7-1 and Example 8, when the microspheres prepared by mixing two or more polylactides, and a mixture of two or more microspheres prepared by different polylactide have both release characteristics of two or more polymers or two or more microspheres. Thus, it was confirmed that it is possible to produce a suitable sustained-release donepezil microsphere prepared by using two or more polylactides and two or more microspheres.

**Claims**

1. A microsphere for donepezil sustained-release injection preparation comprising polylactide and 20 to 40 % (w/w) of donepezil, based on the total weight of the microspheres, wherein the average particle size of the microspheres is 30 to 150 μm, the span value of the microsphere is 1.2 or less, and the polylactide has an intrinsic viscosity of 0.16 to 0.75 dL/g, measured at 0.1% (w/v) concentration in chloroform at 25 °C using a Ubbelohde viscometer wherein when the microsphere is administered to SD rats intramuscularly, the donepezil release is 0 % to 8 % in 24 hours, 20 % to 75 % in 21 days, 80 % to 100 % in 56 days, and the donepezil release in any two weeks from administration to 56 days after administration is 5 % to 65 %.

2. The sustained-release microsphere of claim 1, wherein when 200 mg of the sustained-release donepezil microspheres is suspended in 0.5 mL of distilled water and recovered using a 23 G syringe needle, 80 % (w/w) or more of microspheres are recovered.

3. The sustained-release microsphere of claim 1, wherein the sustained-release microsphere comprises at least two polylactides having different intrinsic viscosities.

4. The sustained-release microsphere of claim 1, wherein the sustained-release microsphere comprises two or more microspheres which comprise a polylactide having a different intrinsic viscosity.

5. A method for preparing donepezil microspheres comprising:

   (a) dissolving donepezil and polylactide polymer in an organic solvent to prepare a donepezil-polylactide solution (dispersed phase);
   (b) adding the donepezil-polylactide solution prepared in step (a) to an aqueous phase containing a surfactant (continuous phase) to prepare an emulsion;
   (c) extracting and evaporating the organic solvent from the dispersed phase in the form of emulsion prepared in the step (b) into the continuous phase to form microspheres; and

(d) recovering the microspheres from the continuous phase containing the produced microspheres of step (c), wherein

the average particle size of the microspheres is 30 to 150 μm, the span value of the microsphere is 1.2 or less, and the polylactide has an intrinsic viscosity of 0.16 to 0.75 dL/g, measured at 0.1% (w/v) concentration in chloroform at 25 °C using a Ubbelohde viscometer

wherein when the microsphere is administered to SD rats intramuscularly, the donepezil release is 0 % to 8 % in 24 hours, 20 % to 75 % in 21 days, 80 % to 100 % in 56 days, and the donepezil release in any two weeks from administration to 56 days after administration is 5 % to 65 %.

6. The method of claim 5, further comprising a sieving process between step (c) and step (d).

7. The method of claim 5, wherein the surfactant in step (b) is one or more selected from the group consisting of methylcellulose, polyvinylpyrrolidone, carboxymethylcellulose, lecithin, gelatin, polyvinyl alcohol, polyoxyethylene sorbitan fatty acid esters, polyoxyethylene castor oil derivatives and a mixture thereof.

[FIG. 1A]

Example 3

[FIG. 1B]

Comparative example 4

Europäisches
Patentamt

European
Patent Office

Office européen
des brevets

# EUROPEAN SEARCH REPORT

Application Number

EP 22 19 4040

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 101 167 697 B (SHANGHAI INST MATERIA MEDICA) 30 March 2011 (2011-03-30) * examples 2,8,11,12,15 * | 1-7 | INV. A61K9/16 A61K9/00 A61K31/445 A61P25/28 |
| A,D | KR 2014 0120496 A (DONG KOOK PHARM CO LTD [KR]) 14 October 2014 (2014-10-14) * paragraphs [0010], [0024] – [0027]; claims 1-15 * | 1-7 | |
| A | Forbes R T ET AL: "Poly(DL-Lactic Acid)" In: "Handbook of Pharmaceutical Excipients", 31 December 2009 (2009-12-31), Pharmaceutical Press, XP55879243, pages 515-517, * the whole document * | 1-7 | |

TECHNICAL FIELDS
SEARCHED (IPC)

A61K

The present search report has been drawn up for all claims

| Place of search | Date of completion of the search | Examiner |
|---|---|---|
| Munich | 27 October 2022 | Benbow, Susanne |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
......................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 4040

This annex lists the patent family members relating to the patent documents cited in the above-mentioned European search report.
The members are as contained in the European Patent Office EDP file on
The European Patent Office is in no way liable for these particulars which are merely given for the purpose of information.

27-10-2022

| Patent document cited in search report | | Publication date | Patent family member(s) | | Publication date |
|---|---|---|---|---|---|
| CN 101167697 | B | 30-03-2011 | NONE | | |
| KR 20140120496 | A | 14-10-2014 | CA | 2908248 A1 | 09-10-2014 |
| | | | CN | 105338966 A | 17-02-2016 |
| | | | EP | 2982367 A1 | 10-02-2016 |
| | | | JP | 2016515612 A | 30-05-2016 |
| | | | KR | 20140120496 A | 14-10-2014 |
| | | | US | 2016022583 A1 | 28-01-2016 |
| | | | WO | 2014163400 A1 | 09-10-2014 |

EPO FORM P0459

For more details about this annex : see Official Journal of the European Patent Office, No. 12/82

**REFERENCES CITED IN THE DESCRIPTION**

**Patent documents cited in the description**

- JP 11315016 A
- KR 1020140120496

- CN 101167697 B

**Non-patent literature cited in the description**

- **PENGCHENG ZHANG et al.** *Biomaterials,* 2007, vol. 28, 1882-1888